# EUROPÄISCHE PATENTSCHRIFT

(11) **EP 3 270 877 B1**
(45) Veröffentlichungstag und Bekanntmachung des Hinweises auf die Patenterteilung: **12.06.2019**
(21) Anmeldenummer: 16706601.8
(22) Anmeldetag: 25.02.2016
(51) Int. Cl.: A61K 8/41, A61Q 1/14, A61K 8/06

(54) **MULTIFUNKTIONSZUBEREITUNG ZUR GESICHTSREINIGUNG**
MULTI-FUNCTIONAL PREPARATION FOR FACIAL CLEANSING
PRÉPARATION MULTIFONCTION POUR LE NETTOYAGE DU VISAGE

(30) Priorität: 16.03.2015 DE 102015204662
(43) Veröffentlichungstag der Anmeldung: 24.01.2018
(73) Patentinhaber: Beiersdorf AG, 20253 Hamburg (DE)
(72) Erfinder: VETTER, Katrin, 23843 Bad Oldesloe (DE); SCHORNSTEIN, Ina, 25421 Pinneberg (DE)
(86) Internationale Anmeldenummer: PCT/EP2016/053983
(87) Internationale Veröffentlichungsnummer: WO 2016/146360

(56) Entgegenhaltungen:
- EP-A1- 2 732 805

## Beschreibung

Die Reinigung des Gesichts ist ein wichtiger Prozess in der täglichen Körperhygiene. In der westlichen Welt ist das Gesicht für die Mitmenschen immer zu sehen und praktisch "die Visitenkarte" des Menschen. Aus diesem Grund bedarf es einer gründlichen Reinigung und Pflege. Dabei müssen Verschmutzungen, die auf der Gesichtshaut und auf angrenzenden Hautarealen nebst den darauf wachsenden Haaren anhaften, entfernt werden. Die Verschmutzungen können eigene Körperprodukte, beispielsweise Talg, Nasensekret, Tränen und Schweiß, aus der Umwelt stammende Verschmutzungen, beispielsweise Schmutz und Staub, oder auch Reste von Kosmetikprodukten beispielsweise Make-Up, Lichtschutzmittel, Mascara und Cremes, sein. Gerade Make-up-Zubereitungen für die Augen, wie beispielsweise Mascaras oder Lidschatten, sollen lange auf der Haut- oder auf Hautanhangspartien, auf die sie aufgetragen wurden, bleiben, nicht verschmieren, sich nicht ausbreiten und nicht übertragbar sein. Dies bedingt, dass es Zubereitungen sind, die vielfach eine Öl- und/oder Fettbasis haben und optional bestimmte Silikonverbindungen aufweisen.

Es ist also wünschenswert, Zubereitungen zur Verfügung zu haben, die die oben aufgeführten Verschmutzungen, insbesondere jedoch Make-up-Produkte, problemlos entfernen können.

Im Stand der Technik sind geeignete Zubereitungen bereits zu finden. Es handelt sich oft um zweiphasige Zubereitungen aus einem bestimmten Anteil einer Öl- und einer Wasserphase.

Das Produkt "Démaquillant Bi-Phase, Barbara Gould der Firma Santé Beauté wurde ab März 2014 in Frankreich verkauft. (Mintel-Datenbank Eintragungsnummer-Nummer: 2355487). Das Produkt ist ein Zwei-Phasen-Produkt und wird zur Make-up-entfernung eingesetzt. Die Konservierung erfolgt u.a. mit Benzyl Alkohol.

Die Firma Unilever vertreibt seit Februar 2012 das Produkt Bio-Hydrante unter der Marke Pond's (Mintel-Datenbank Eintragungsnummer-Nummer: 1725845). Es handelt sich um ein zwei-phasiges Produkt mit einer Öl- und Wasserphase. Die Konservierung erfolgt mit Benza-Ikonium Chloride.

EP 0827736 A1 offenbart eine biphasige Zubereitung, um Make-up-Zubereitungen zu entfernen, Diese Make-up-Zubereitungen zeichnen sich dadurch aus, dass sie nicht übertragen werden (non-transfert) und einen langen Halt haben (longue tenue). Die biphasigen Zubereitungen werden dazu verwendet derartige Make-up-Zubereitungen, insbesondere jedoch Lippenstiftzubereitungen, zu entfernen. Im Zusammenhang mit der Lippenstiftentfernung werden die biphasigen Zubereitungen gemäß EP 0827736 als frisch empfunden.

EP 0974333 beschreibt biphasige Zusammensetzungen zur Reinigung der Haut und zur Make-up-Entfernung. Die Zusammensetzungen zeichnen sich durch das Vorhandensein eines bestimmten Konservierungsmittels aus, nämlich Polyaminopropyl Biguanide.

EP 2732805 offenbart eine zweiphasige Zubereitung umfassend eine Öl- und eine Wasserphase und Benzethoniumchlorid. Diese Zubereitung wird als Reinigungszubereitung, insbesondere als Make-up-Entferner, verwendet.

Alle die Zubereitungen und Produkte erfüllen die Aufgabe, Make-up von der Haut und den Hautanhangsgebilden des Gesichts zu entfernen.

Dennoch waren die Anwender mit den Produkten des Standes der Technik nicht zufrieden. Es wurde bemängelt, dass die Zubereitungen zu "ölig" seien und dass die Haut nach Anwendung derartiger Zubereitungen einen Ölfilm aufweise und sich ölig anfühle. Zudem bestand der Wunsch nach Zubereitungen zur Gesichtsreinigung und Make-up-Entfernung, die ein frisches Hautgefühl vermitteln, also erfrischend und tonisierend wirken.

Kundenbefragungen zeigten, dass die Anwender häufig den Ölfilm, der nach der Verwendung der Produkte des Standes der Technik auf der Haut verbleibt, in einem zusätzlichen Reinigungsschritt wieder entfernen. Ebenso wurde in anderen Fällen im Anschluss an die Verwendung von Produkten des Standes der Technik ein Gesichtswasser auf die Haut aufgetragen um die Haut zu erfrischen und zu tonisieren. Es bestand also ein vielfacher Wunsch nach einem 3-in-1-Produkt oder Multifunktionsprodukt, das alle Funktionen, Gesichtsreinigung, Make-up-Entfernung und Erfrischung/Tonisierung, in einem Produkt erfüllt.

Es bestand daher die Aufgabe, Zubereitungen zur Verfügung zu stellen, die eine Gesichtsreinigung und Make-up-Entfernung bewirken und darüber hinaus als Gesichtswasser eine Erfrischung und Tonisierung der Haut erzielen.

Darüber hinaus sollten Zubereitungen zur Verfügung gestellt werden, die keine Silikon-haltigen Verbindungen enthalten.

Gelöst wurde diese Aufgabe durch zwei-phasige Gesichtsreinigungszubereitungen, umfassend eine Öl- und Wasserphase und Benzethoniumchlorid, wobei die Ölphase wenigstens Isohexadecane und/oder C₁₅₋₁₉-Alkane enthält und wobei die Zubereitungen keine Silikon-haltigen Verbindungen enthalten.

Ebenso Gegenstand der vorliegenden Erfindung ist die Verwendung von zwei-phasige Gesichtsreinigungszubereitungen, umfassend eine Öl- und Wasserphase und Benzethoniumchlorid, wobei die Ölphase wenigstens Isohexadecane und/oder C₁₅₋₁₉-Alkane enthält und wobei die Zubereitungen keine Silikon-haltigen Verbindungen enthalten, zur gleichzeitigen Gesichtsreinigung, Make-up-Entfernung und tonisierenden Gesichtserfrischung.

Weiterhin Gegenstand der vorliegenden Erfindung sind zwei-phasige Gesichtsreinigungszubereitungen zur Anwendung im Gesicht, wobei die Zubereitungen vor Gebrauch durch Schütteln vermischt werden.

Erfindungsgemäß vorteilhaft ist es, wenn sowohl die Wasserphase als auch die Ölphase transparent sind. Die Wasserphase kann aber Farbstoffe enthalten und erscheint dann durchscheinend farbig. Ebenso kann die Ölphase Farbstoffe enthalten. Die Ölphase erscheint dann durchscheinend farbig. Darüber hinaus können die Wasserphase und die Ölphase Farbstoffe enthalten. Beide Phasen erscheinen dann durchscheinend farbig.

Das Volumenverhältnis von Ölphase zu Wasserphase beträgt 20 zu 80 bis 40 zu 60 Vol.-%, bevorzugt 25 zu 75 bis 35 zu 65 Vol.-%, besonders bevorzugt 30 zu 70 Vol.-%, bezogen auf das Gesamtvolumen der Zubereitung.

Die Ölphase der erfindungsgemäßen Zubereitungen muss eine bestimmte Zusammensetzung aufweisen, um die oben genannte Aufgabe zu lösen. Es ist wichtig, dass wenigstens Isohexadecan und/oder ein C₁₅₋₁₉-Alkan in der Ölphase enthalten sind. Darüber hinaus kann ein weiterer oder können weitere Bestandteile enthalten sein, die aus der Gruppe Isododecan und Coco-Caprylat/Caprat gewählt werden können. Isohexadecane ist beispielsweise erhältlich unter der Handelsbezeichnung Arlamol HD von der Firma Croda, C₁₅₋₁₉-Alkane ist beispielsweise erhältlich unter der Handelsbezeichnung Gemseal 40 von der Firma Total Fluids.

Es ist erfindungsgemäß bevorzugt, wenn die erfindungsgemäßen Zubereitungen kein Isopropylpalmitat enthalten.

Ebenso ist es erfindungsgemäß bevorzugt, wenn die Zubereitungen kein C₁₃₋₁₆- Isoparaffin, enthalten.

Bevorzugt ist auch, wenn die erfindungsgemäßen Zubereitungen kein Isopropylpalmitat und kein C₁₃₋₁₆- Isoparaffin enthalten.

Die erfindungsgemäßen Zubereitungen enthalten keine Silikon-haltigen Verbindungen, das bedeutet, dass den erfindungsgemäßen Zubereitungen keine Silikon-haltigen Verbindungen zugesetzt werden und auch keine Silikon-haltigen Verbindungen mit anderen Zubereitungsbestandteilen eingeschleppt werden. Silikon-haltige Verbindungen zeichnen sich durch das Vorhandensein von Silicium- und Sauerstoffatomen aus, die miteinander verknüpft sind und eine Vielzahl verschiedener Verbindungen ausbilden können.

Die einzelnen Bestandteile der Ölphase liegen mit einem Gehalt von 0,05 bis 40 Gew.-%, bevorzugt 0,5 bis 35 Gew.-% vor, ganz besonders von 0,75 bis 25 Gew.-%, bezogen auf das Gesamtgewicht der Zubereitung vor. In Sinne der vorliegenden Erfindung werden unter einzelnen Bestandteilen der Ölphase diejenigen Bestandteile verstanden, die als erfindungsgemäße Bestandteile der Ölphase beschrieben sind oder in den Beispielen mit der Funktionsbezeichnung Lipid versehen sind.

Die erfindungsgemäßen Zubereitungen sind Zwei-Phasen-Zubereitungen. Sie werden vor Gebrauch durch Schütteln vermischt. Nach dem Gebrauch entmischen sich die Phasen innerhalb kurzer Zeit. Das Vorliegen zweier getrennter Phasen kann dem Anwender etwa durch unterschiedliche Färbung der Phasen angezeigt werden.

Im Bereich der Reinigungsprodukte hat sich gezeigt, dass zweiphasige Systeme eine bessere Reinigungsleistung haben, besonders bei der Entfernung von wasserfestem Make-up. Es wird die gut reinigende Wirkung von Ölen mit Wasser kombiniert.

Um ein Zwei-Phasenprodukt zu bilden, bedarf es einer Phase mit Wasser und einer Phase mit Öl. Im Ruhezustand sind diese beiden Phasen idealerweise optisch klar voneinander getrennt zu erkennen. Durch die unterschiedlichen Dichten vermischen sich diese beiden Phasen nicht selbstständig miteinander und bilden dadurch ein horizontal getrenntes zwei-phasiges System aus. Durch das Einbringen von Energie, z.B. durch Schütteln, kann man die Phasen kurzzeitig miteinander vermischen. Aufgrund des Dichteunterschiedes und einer hohen Grenzflächenspannung (GFS) von Öl gegenüber Wasser kommt es allerdings danach wieder zu einer sehr schnellen Auftrennung in die zwei Phasen.

Um eine Verwendung zwei-phasiger Produkte zu ermöglichen, sollte die Trenngeschwindigkeit und insbesondere der Trennungsstart vorteilhafterweise verlangsamt werden, da man ansonsten nur eine Phase bzw. zum größten Teil nur eine Phase entnehmen und anwenden kann. Dies bedeutet, dass bei umgehender Phasentrennung nach dem Schütteln sich mitunter nur eine Phase entnehmen lässt und die Reinigung z.B. nur mit Wasser erfolgt. Die Folge ist eine geringere Reinigungsleistung, da die reinigende Wirkung des Öls fehlt.

Durch den Zusatz von Benzethonium Chlorid kann in Zweiphasenzubereitungen eine Verlängerung des Phasentrennungsstartes nach dem Vermischen der beiden Phasen erreicht werden.

Der Trennungsstart ist der Zeitpunkt, ab dem die Trennung der beiden Phasen optisch erkennbar wird und noch keine vollständig getrennten Phasen vorliegen. Die Trennungszeit kann durch den Benzethoniumchlorid-Zusatz verlängert werden, in der Regel um 20 bis 25 Sekunden, in Einzelfällen bis 15 Minuten. Hierdurch wird die Verwendung von zweiphasigen Zubereitungen für den Konsumenten erst möglich. Der Anwender kann nun in ausreichend verlängerter Zeit das Zwei-Phasenprodukt anwenden ohne befürchten zu müssen, dass die Phasen schon wieder getrennt vorliegen.

Die Trennung in zwei Phasen mit klarer Grenzfläche ist in der Regel nach etwa 15 Minuten ungefähr abgeschlossen; die Phasen können dann noch leicht trübe sein, abhängig davon wie stark und lange geschüttelt wurde. Nach < 12 Stunden sind beide Phasen wieder klar.

Die Offenbarung zu Benzethonium-haltigen Zwei-Phasen-Zubereitungen ist im Dokument DE 102012219641 zu finden. Die Lehre dieses Dokuments bezüglich Benzethonium-haltiger Zwei-Phasen-Zubereitungen ist hiermit in die Offenbarung dieser Anmeldung eingeschlossen.

Benzethonium Chlorid liegt in den erfindungsgemäßen Zubereitungen mit einem Gehalt von 0,01 bis 1 Gew.-%, insbesondere 0,03 bis 0,05 Gew.-%, bezogen auf das Gesamtgewicht der Zubereitung, vor.

Bei Zwei-phasigen Produkten ohne Benzethonium Chlorid kann der Zusatz von nichtionischen Tensiden bewirken, dass es nach dem Mischen der beiden Phasen zu optischen Phänomenen kommt, wie z.B. Schaumbildung an der Grenzfläche, emulgierte Phasen oder Phasenanteile, Trübungen einer oder beider Phasen.

Durch den Einsatz von Benzethonium Chlorid in Zwei-phasigen Zubereitungen ist es möglich wenigstens ein nichtionisches Tensid, ausgewählt aus der Gruppe Caprylyl/Capryl Glucoside, Decyl Glucoside oder auch Polyglyceryl-10 Laurate, in die erfindungsgemäßen Zubereitungen einzuarbeiten, ohne dass es zu den oben genannten Problemen kommt.

Das wenigstens eine nichtionische Tensid liegt mit einem Gehalt von 0,01 bis 2 Gew.-%, insbesondere mit 0,03 bis 0,05 Gew.-%, bezogen auf das Gesamtgewicht der Zubereitung, vor.

Weiterhin führt der Einsatz von Benzethonium Chlorid in zwei-phasigen Zubereitungen dazu, dass der Anteil an weiteren Konservierungsmitteln, beispielsweise Phenoxyethanol, an Trisodium Trisodium-Ethylenediaminetetraacetic acid (EDTA) und an Natriumchlorid verringert werden kann.

Demgemäß umfassen die erfindungsgemässen Zubereitungen vorteilhaft Natriumchlorid und Trisodium-Ethylenediaminetetraacetic acid sowie gegebenenfalls ein oder mehrere Wirkstoffe, Parfüme, Farbstoffe und/oder Hautbefeuchtungsmittel. Bevorzugte Hautbefeuchtungsmittel sind Glycerin und/oder Glyceryl Glucosid. Vorteilhaft wird Glycerin und/oder Glyceryl Glucosid der Zubereitung zu einem Anteil bis zu jeweils 5 Gew.-% zugesetzt, bezogen auf das Gesamtgewicht der Zubereitung.

Als öllösliche Farbstoffe werden insbesondere Cl 61565 (1,4-bis(p-tolylamino)anthraquinone) und Cl 60725 (1-Hydroxy-4-(p-toluidino)anthrachinon) sowie als wasserlöslicher Farbstoff insbesondere Cl 16035 (FD&C Red 40; 6-Hydroxy-5-((6-methoxy-4-sulfo-m-tolyl)azo)-2-naphthalenesulfonic acid disodium salt) gewählt.

Im Sinne der vorliegenden Erfindung bedeutet der Ausdruck "Substanz XY nicht enthalten" bedeutet, dass den erfindungsgemäßen Zubereitungen diese Substanz nicht zugesetzt wird und auch nicht mit anderen Bestandteilen der jeweiligen Zubereitungen eingeschleppt wird.

Zu den Hautanhangsgebilden zählen die Haupt- und Körperhaare sowie insbesondere die Finger- und Fußnägel.

Um die Wirksamkeit von ausgewählten erfindungsgemäßen Zubereitung zu untersuchen, wurden zwei Zubereitungen hergestellt. Die Gesichtsreinigungszubereitungen, SHAKE-569 oder F58 und SHAKE-570 oder L12, sind beides erfindungsgemäßen Zubereitungen, die sich in der Zusammensetzung der Ölphase unterscheiden. SHAKE-570 enthält Isohexadecane, während SHAKE-569 C₁₅₋₁₉-Alkane enthält.

| INCI Bezeichnung | Funktion | SHAKE-570 (erfindungsgemäßes Beispiel) | SHAKE-569 (erfindungsgemäßes Beispiel) |
|---|---|---|---|
| Cl 16035 | Farbstoff | 0,09 | 0,09 |
| Isododecane | Lipid | 20,00 | 22,00 |
| C₁₅₋₁₉ Alkane | Lipid | | 6,00 |
| Coco-Caprylate/Caprate | Lipid | 1,00 | 2,00 |
| Isohexadecane | Lipid | 9,00 | |
| Glycerin + Aqua (99%ig in H₂O) | Hautbefeuchter | 5,00 | 3,50 |
| Benzethonium Chloride | Konservierungsmittel | 0,04 | 0,04 |
| Phenoxyethanol | Konservierungsmittel | 0,30 | ,030 |
| Natrium Chlorid | Verdicker | 0,50 | 0,35 |
| Wasser | | 63,05 | 64,76 |
| Aqua + Trisodium EDTA (20%ig in H₂O) | Chelator | 0,95 | 0,90 |
| Caprylyl/Capryl Glucoside + Aqua (66%ig in H₂O) | Tensid | 0,07 | 0,06 |
| Summe | | 100,00 | 100,00 |

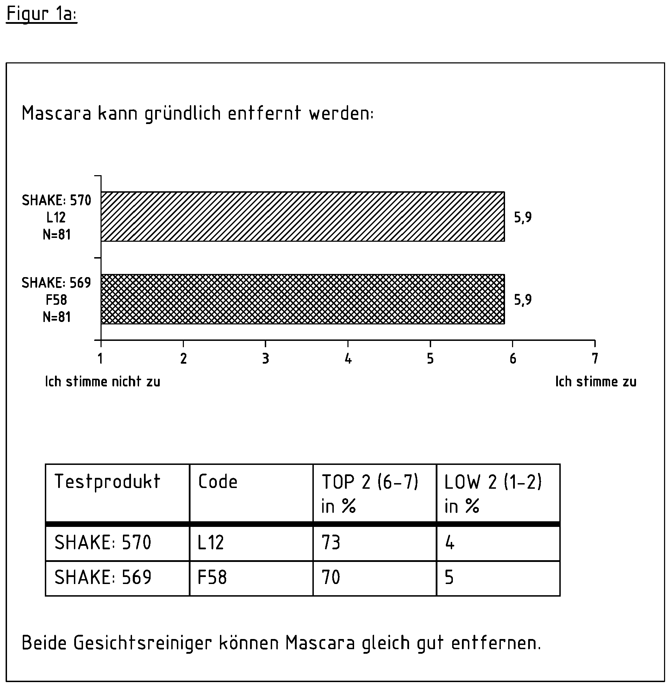

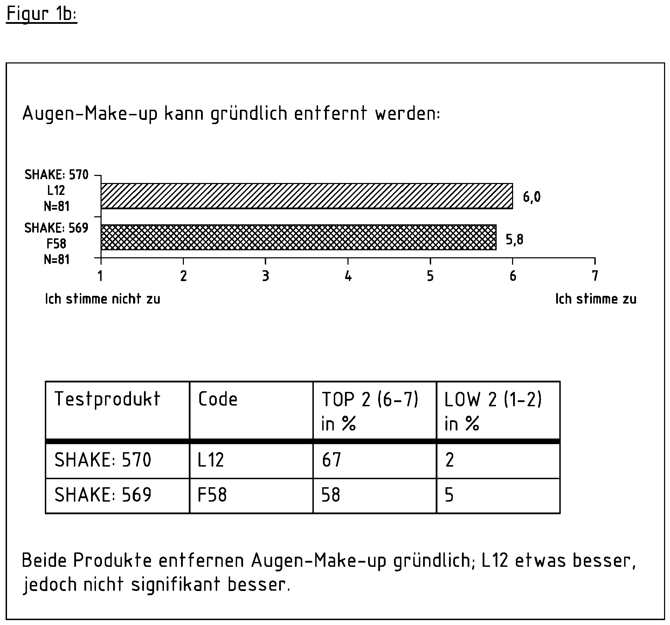

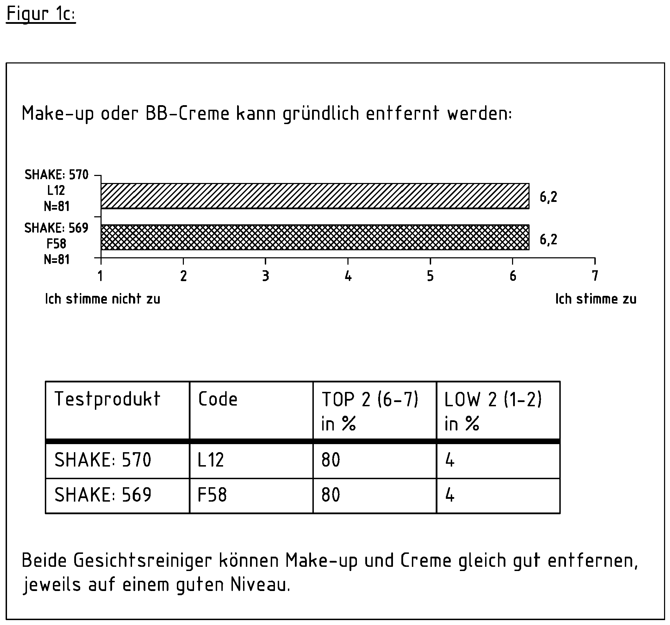

Die beiden genannten Gesichtsreinigungszubereitungen wurden in einem Home-in-Use-Test untersucht. Dazu erhielten 100 Frauen im Alter von 25 bis 56 (Durchschnittsalter 44,77 Jahre) die zwei Gesichtsreiniger. Die Produkte sollten jeweils 7 Tage angewendet werden. Anschließend wurde ein Fragebogen beantwortet. Von den 100 Frauen gaben 81 den Fragebogen zurück. In den Abbildungen 1a bis 3b sind Ergebnisse dargestellt, die für die Beurteilung, ob die Aufgabe, die der Erfindung zu Grunde lag, gelöst wurde, relevant sind. Für die statische Auswertung wurden Mittelwerte, absolute und relative Häufigkeiten und "Top Boxes" und "Low Boxes" ermittelt. Die Werte zu den Top Boxes geben an, wie häufig die besten Bewertungen (7 und 6, 7 bedeutet "ich stimme zu") vergeben wurden. Entsprechend gilt für die Low Boxes, wie häufig die Beurteilung 1 und 2 (1 bedeutet "ich stimme nicht zu") angegeben wurden.

Die Figuren 1a bis 1c stellen die Ergebnisse zur Beurteilung der Gesichtsreiniger in Bezug auf die Wirkung als Make-Up-Entferner dar. 90 % der Probandinnen verwenden Augen-Make-up, 10 % hingegen nicht. BB-Creme wird von 96 % der Probandinnen verwendet, von 4 % jedoch nicht. Mit beiden Gesichtsreinigern kann Make-Up, in Form von Mascara, Augen-Make-Up und BB-Creme, entfernt werden. Augen-Make-up kann mit dem Gesichtsreiniger gemäß Beispiel SHAKE-570 besser entfernt werden, jedoch nicht signifikant besser.

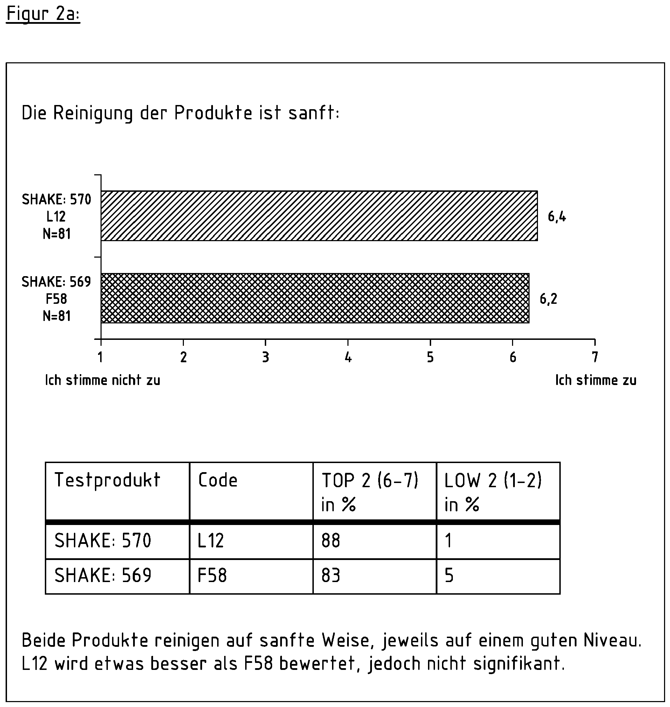

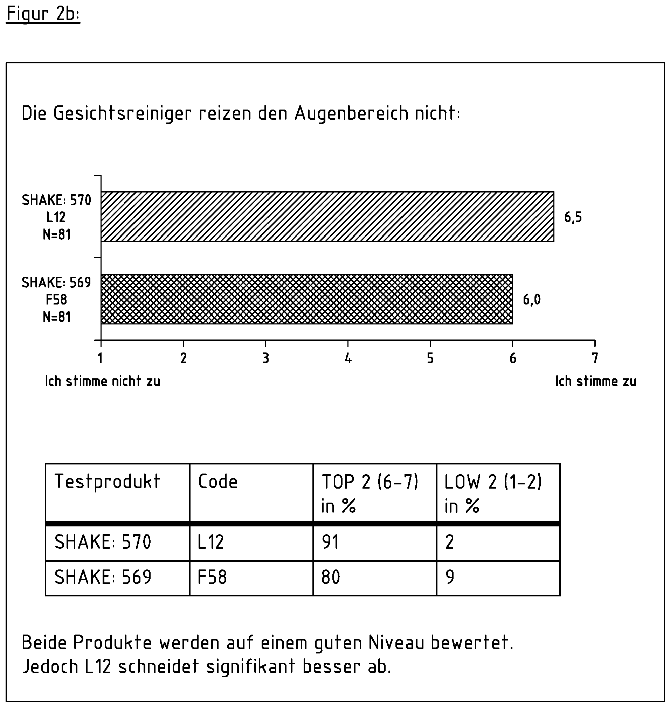

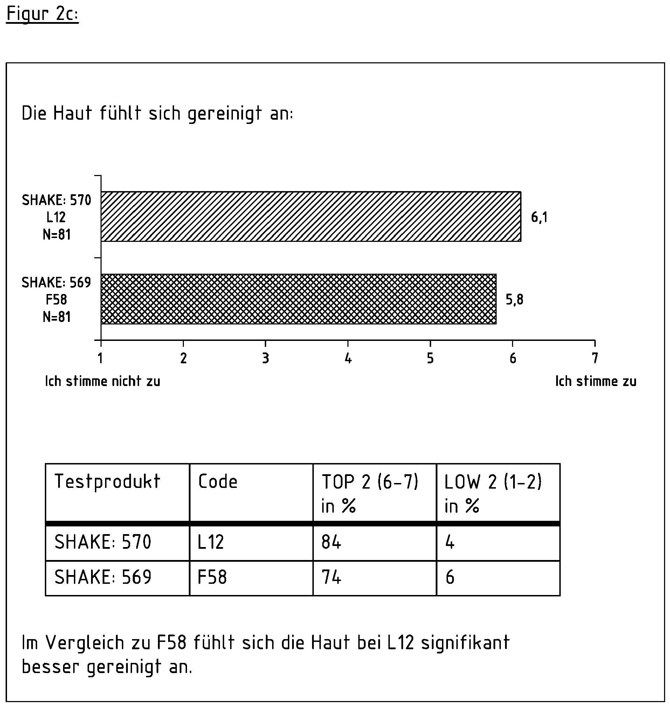

Die Darstellungen der Figuren 2a bis 2c enthalten Informationen zur Reinigungswirkung der Gesichtsreiniger. In der Reinigungsleistung (Figur 2c) ist der Gesichtsreiniger SHAKE-570 signifikant besser als derjenige gemäß Beispiel SHAKE-569. Die Figuren 2a und 2b zeigen die Ergebnisse in Bezug auf die Sanftheit der Reinigung (Figur 2a) und Wirkung auf den Augenbereich (Figur 2b). Beide Gesichtsreiniger reizen den Augenbereich nicht, das Produkt gemäß Beispiel SHAKE 570 wird signifikant besser bewertet als das Produkt gemäß Beispiel SHAKE-569. Hinsichtlich der Sanftheit in der Reinigung schneidet das Produkt SHAKE-570 ebenfalls besser ab, als das Produkt gemäß SHAKE-569. Alle Ergebnisse in Bezug auf die Reinigungswirkung zeigen jedoch für beide Produkte eine ausgesprochen gute Reinigungswirkung.

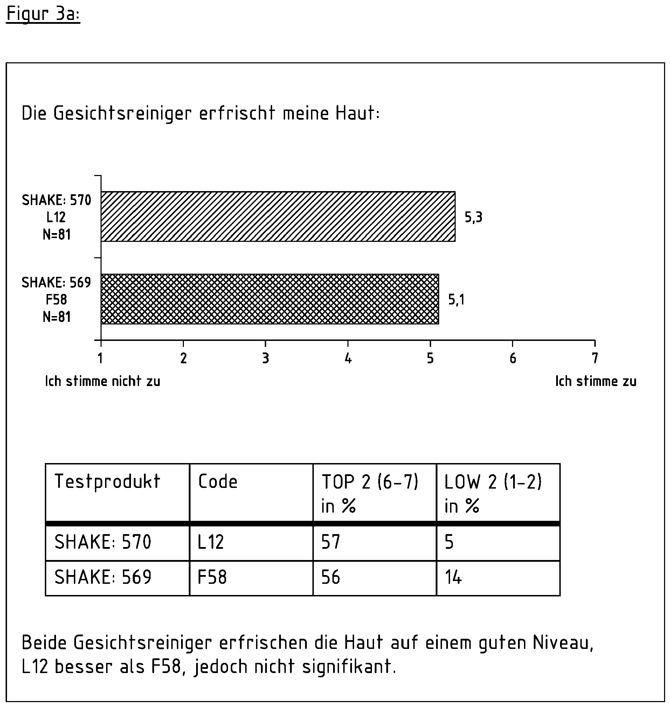

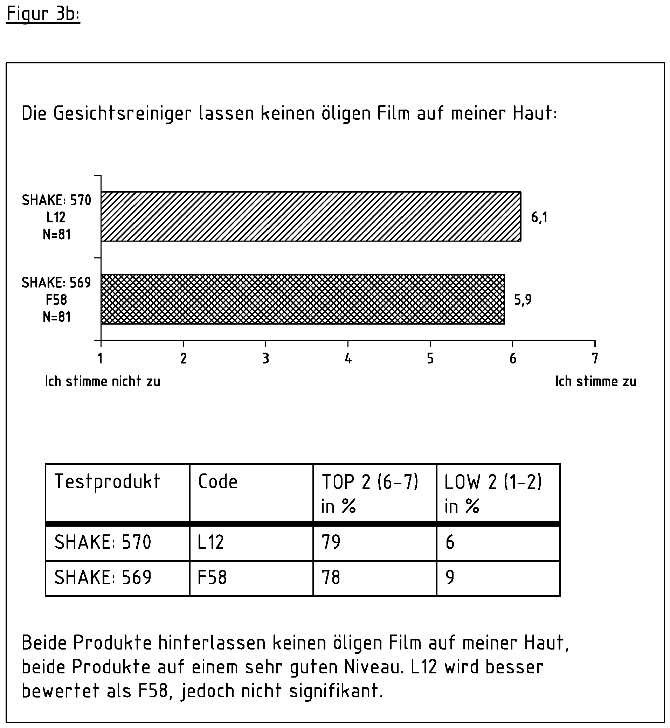

Die Abbildungen 3a und 3b zeigen die Ergebnisse in Bezug auf die erfrischende Wirkung der Gesichtsreiniger und das Auftreten eines öligen Gefühls auf der Gesichtshaut nach der Anwendung. In beiden Fällen schneidet die Zubereitung gemäß Beispiel SHAKE-570 besser ab als die diejenige gemäß Beispiel SHAKE-569. Der Unterschied ist jedoch nicht signifikant.

Beide erfindungsgemäßen Produkte zeigen in der Make-up-Entfernung eine vergleichbar sehr gute Leistung. In Bezug auf die Reinigungsleistung, die Sanftheit der Reinigung und die Milde im Augenbereich wird das Produkt gemäß SHAKE-570 besser bewertet. Beide erfindungsgemäßen Produkte zeigen hinsichtlich der beurteilten Kriterien insgesamt eine sehr gute Leistung. Auch hinsichtlich der Erfrischung der Gesichtshaut und des Fehlen eines öligen Films nach der Anwendung sind die Beurteilungen für das Produkt gemäß SHAKE-570 besser, aber auch das Produkt gemäß Beispiel SHAKE-569 schneidet gut ab. Hieraus kann abgeleitet werden, dass das Produkt SHAKE-570 von den Anwendern etwas besser beurteilt wurde als das Produkt SHAKE-569. Beide Produkte erhalten aber insgesamt gute bis sehr gute Beurteilungen und sind daher als "Multifunktionsprodukte" oder 3-in-1-Produkte geeignet.

Die erfindungsgemäßen Zubereitungen, insbesondere die Zubereitungen gemäß SHAKE-570 und SHAKE-569, sind Zubereitungen, die zu überaus zufriedenstellenden Ergebnissen in Bezug auf die Entfernung von Make-up und Augen-Make-up führen. Ebenso überaus zufriedenstellend ist die Reinigungsleistung der erfindungsgemäßen Zubereitungen, verbunden mit einer als angenehm empfundenen Sanftheit der Reinigung. Darüber hinaus ist es besonders zufriedenstellend, dass es gelungen ist, ein öliges Gefühl nach der Anwendung zu vermeiden, wohingegen sich ein Gefühl von Frische auf der Haut nach der Anwendung einstellt.

### Beispiele:

Alle Angaben in der vorliegenden Anmeldung sind Angaben in Gew.-%, sofern nicht ausdrücklich andere Einheiten angegeben sind.

Die Angaben in den unten stehenden Beispiele geben die Rohstoffgehalte an. Diese entsprechen den Aktivgehalten, sofern nicht bei der INCI-Bezeichnung der jeweilige prozentuale Aktivgehalt angegeben ist.

**Beispielrezepturen:**

| **INCI Bezeichnung** | **Funktion** | **Beispiel 1** | **Beispiel 2** | **Beispiel 3** |
|---|---|---|---|---|
| CI 16035 | Farbstoff | | 0,09 | |
| CI 61565 | Farbstoff | 0,0003 | | 0,0002 |
| CI 60725 | Farbstoff | 0,0007 | | 0,0008 |
| Isododecane | Lipid | 18,00 | 21,00 | 15,00 |
| C₁₅₋₁₉ Alkane | Lipid | 10,00 | | 12,00 |
| Coco-Caprylate/Caprate | Lipid | 2,00 | 4,00 | 3,00 |
| Isohexadecane | Lipid | | 5,00 | |
| Glycerin + Aqua (99%ig in H₂O) | Hautbefeuchter | 4,20 | 5,00 | 3,5 |
| Benzethonium Chloride | Konservierungsmittel | 0,04 | 0,04 | 0,04 |
| Phenoxyethanol | Konservierungsmittel | 0,30 | 0,30 | 0,30 |
| Natrium Chlorid | Verdicker | 0,2 | 0,5 | 0,35 |
| Wasser | | 64,299 | 63,05 | 64,849 |
| Aqua + Trisodium EDTA (20%ig in H₂O) | Chelator | 0,90 | 0,95 | 0,90 |
| Caprylyl/Capryl Glucoside + Aqua (60%ig in H₂O) | Tensid | 0,06 | 0,070 | 0,06 |
| Summe | | 100,00 | 100,00 | 100,00 |

## Patentansprüche

1. Zwei-phasige Gesichtsreinigungszubereitungen, umfassend eine Öl- und Wasserphase und Benzethoniumchlorid, wobei die Ölphase wenigstens Isohexadecane und/oder C₁₅₋₁₉-Alkane enthält und wobei die Zubereitungen keine Silikon-haltigen Verbindungen enthalten.

2. Zwei-phasige Gesichtsreinigungszubereitungen nach Anspruch 1, **dadurch gekennzeichnet, dass** das Volumenverhältnis von Ölphase zu Wasserphase 20 zu 80 bis 40 zu 60 Vol.-%, bevorzugt 25 zu 75 bis 35 zu 65 Vol.-%, besonders bevorzugt 30 zu 70 Vol.-%, bezogen auf das Gesamtvolumen der Zubereitung, beträgt.

3. Zwei-phasige Gesichtsreinigungszubereitungen nach Anspruch 1 und/oder 2, **dadurch gekennzeichnet, dass** die Ölphase wenigstens einen weiteren Bestandteil, ausgewählt aus der Gruppe Isododecan und Coco-Caprylat/Caprat, enthält.

4. Zwei-phasige Gesichtsreinigungszubereitungen nach wenigstens einem der vorstehenden Ansprüche, **dadurch gekennzeichnet, dass** die einzelnen Bestandteile der Ölphase mit einem Gehalt von 0,05 bis 40 Gew.-%, bevorzugt 0,5 bis 35 Gew.-% vor, ganz besonders von 0,75 bis 25 Gew.-%, bezogen auf das Gesamtgewicht der Zubereitung vorliegen.

5. Zwei-phasige Gesichtsreinigungszubereitungen nach wenigstens einem der vorstehenden Ansprüche, **dadurch gekennzeichnet, dass** kein Isopropylpalmitat enthalten ist.

6. Zwei-phasige Gesichtsreinigungszubereitungen nach wenigstens einem der vorstehenden Ansprüche, **dadurch gekennzeichnet, dass** die Wasserphase und die Ölphase transparent sind.

7. Zwei-phasige Gesichtsreinigungszubereitungen nach wenigstens einem der vorstehenden Ansprüche, **dadurch gekennzeichnet, dass** die Wasserphase und/oder die Ölphase jeweils farbig durchscheinend sind.

8. Zwei-phasige Gesichtsreinigungszubereitungen nach wenigstens einem der vorstehenden Ansprüche, **dadurch gekennzeichnet, dass** Benzethonium Chlorid mit einem Gehalt von 0,01 bis 1 Gew.-%, insbesondere 0,03 bis 0,05 Gew.-%, bezogen auf das Gesamtgewicht der Zubereitung, vorliegt.

9. Zwei-phasige Gesichtsreinigungszubereitungen nach wenigstens einem der vorstehenden Ansprüche, **dadurch gekennzeichnet, dass** nichtionische Tenside ausgewählt aus der Gruppe Caprylyl/Capryl Glucoside, Decyl Glucoside und Polyglyceryl-10 Laurate enthalten sind.

10. Zwei-phasige Gesichtsreinigungszubereitungen nach wenigstens einem der vorstehenden Ansprüche **dadurch gekennzeichnet, dass** die nichtionische Tenside mit einem Gehalt von 0,01 bis 2 Gew.-%, insbesondere mit 0,03 bis 0,05 Gew.-%, bezogen auf das Gesamtgewicht der Zubereitung, vorliegen.

11. Verwendung von zwei-phasigen Gesichtsreinigungszubereitungen gemäß Anspruch 1 zur gleichzeitigen Gesichtsreinigung, Make-up-Entfernung und tonisierenden Gesichtserfrischung.

## Claims

1. Biphasic facial cleansing preparations comprising an oil and water phase and benzethonium chloride, wherein the oil phase comprises at least isohexadecanes and/or C₁₅₋₁₉-alkanes and wherein the preparations do not comprise any silicone-containing compounds.

2. Biphasic facial cleansing preparations according to Claim 1, **characterized in that** the ratio by volume of oil phase to water phase is 20:80 to 40:60 % by volume, preferably 25:75 to 35:65 % by volume, particularly preferably 30:70 % by volume, based on the total volume of the preparation.

3. Biphasic facial cleansing preparations according to Claim 1 and/or 2, **characterized in that** the oil phase comprises at least one further constituent selected from the group of isododecane and coco-caprylate/caprate.

4. Biphasic facial cleansing preparations according to at least one of the preceding claims, **characterized in that** the individual constituents of the oil phase are present at a content of 0.05 to 40% by weight, preferably 0.5 to 35% by weight, especially preferably from 0.75 to 25% by weight, based on the total weight of the preparation.

5. Biphasic facial cleansing preparations according to at least one of the preceding claims, **characterized in that** no isopropyl palmitate is present.

6. Biphasic facial cleansing preparations according to at least one of the preceding claims, **characterized in that** the water phase and the oil phase are transparent.

7. Biphasic facial cleansing preparations according to at least one of the preceding claims, **characterized in that** the water phase and/or the oil phase are in each case colouredly translucent.

8. Biphasic facial cleansing preparations according to at least one of the preceding claims, **characterized in that** benzethonium chloride is present at a content of 0.01 to 1% by weight, especially 0.03 to 0.05% by weight, based on the total weight of the preparation.

9. Biphasic facial cleansing preparations according to at least one of the preceding claims, **characterized in that** non-ionic surfactants selected from the group of caprylyl/capryl glucoside, decyl glucoside and polyglyceryl-10 laurate are present.

10. Biphasic facial cleansing preparations according to at least one of the preceding claims, **characterized in that** the non-ionic surfactants are present at a content of 0.01 to 2% by weight, especially 0.03 to 0.05% by weight, based on the total weight of the preparation.

11. Use of biphasic facial cleansing preparations according to Claim 1 for simultaneous facial cleansing, make-up removal and toning facial freshening.

## Revendications

1. Préparations à deux phases de nettoyage du visage, comprenant une phase huileuse et une phase aqueuse et du chlorure de benzéthonium, la phase huileuse contenant au moins de l'isohexadécane et/ou des C₁₅₋₁₉-alcanes et les préparations ne contenant pas de composés contenant de la silicone.

2. Préparations à deux phases de nettoyage du visage selon la revendication 1, **caractérisées en ce que** le rapport volumique de la phase huileuse à la phase aqueuse est de 20:80 à 40:60% en volume, de préférence de 25:75 à 35:65% en volume, de manière particulièrement préférée de 30:70% en volume, par rapport au volume total de la préparation.

3. Préparations à deux phases de nettoyage du visage selon la revendication 1 et/ou 2, **caractérisées en ce que** la phase huileuse contient au moins un constituant, choisi dans le groupe formé par l'isododécane et le caprylate/caprate de coco.

4. Préparations à deux phases de nettoyage du visage selon au moins l'une quelconque des revendications précédentes, **caractérisées en ce que** les différents constituants de la phase huileuse sont présents en une teneur de 0,05 à 40% en poids, de préférence de 0,5 à 35% en poids, tout particulièrement de 0,75 à 25% en poids, par rapport au poids total de la préparation.

5. Préparations à deux phases de nettoyage du visage selon au moins l'une quelconque des revendications précédentes, **caractérisées en ce qu'**elles ne contiennent pas de palmitate d'isopropyle.

6. Préparations à deux phases de nettoyage du visage selon au moins l'une quelconque des revendications précédentes, **caractérisées en ce que** la phase aqueuse et la phase huileuse sont transparentes.

7. Préparations à deux phases de nettoyage du visage selon au moins l'une quelconque des revendications précédentes, **caractérisées en ce que** la phase aqueuse et/ou la phase huileuse sont chacune transparentes colorées.

8. Préparations à deux phases de nettoyage du visage selon au moins l'une quelconque des revendications précédentes, **caractérisées en ce que** le chlorure de benzéthonium est présent en une teneur de 0,01 à 1% en poids, en particulier de 0,03 à 0,05% en poids, par rapport au poids total de la préparation.

9. Préparations à deux phases de nettoyage du visage selon au moins l'une quelconque des revendications précédentes, **caractérisées en ce qu'**elles contiennent des tensioactifs non ioniques choisis dans le groupe formé par le glucoside de caprylyle/capryle, le glucoside de décyle et le laurate de polyglycéryle-10.

10. Préparations à deux phases de nettoyage du visage selon au moins l'une quelconque des revendications précédentes, **caractérisées en ce que** les tensioactifs non ioniques sont présents en une teneur de 0,01 à 2% en poids, en particulier de 0,03 à 0,05% en poids, par rapport au poids total de la préparation.

11. Utilisation de préparations à deux phases de nettoyage du visage selon la revendication 1 pour, simultanément, nettoyer le visage, éliminer le maquillage et rafraîchir le visage de manière tonique.
